# FASCICULE DE BREVET EUROPEEN

(11) **EP 2 046 356 B1**
(45) Date de publication et mention de la délivrance du brevet: **06.10.2010**
(21) Numéro de dépôt: 07823291.5
(22) Date de dépôt: 17.07.2007
(51) Int. Cl.: A61K 36/483, A61K 8/97, A61P 3/00, A61Q 19/06

(54) **UTILISATION D'UN EXTRAIT DE GLEDITSIA EN COSMÉTIQUE ET EN DERMATOLOGIE**
VERWENDUNG EINES GLEDITSIA-EXTRAKTS FÜR KOSMETIK UND DERMATOLOGIE
USE OF AN EXTRACT OF GLEDITSIA IN COSMETICS AND IN DERMATOLOGY

(30) Priorité: 20.07.2006 FR 0606609
(43) Date de publication de la demande: 15.04.2009
(73) Titulaire: Laboratoire Nuxe, 75010 Paris (FR)
(72) Inventeur: FIORENTINO, Julia, 75013 Paris (FR); LECLERE, Jacques, 45500 Saint-Gondon (FR)
(74) Mandataire: L'Helgoualch, Jean
(86) Numéro de dépôt international: PCT/FR2007/001224
(87) Numéro de publication internationale: WO 2008/009813

(56) Documents cités:
- GB-A- 1 065 910
- YAMAMOTO YUKIKO ET AL: "Improved hypolipidemic effects of xanthan gum-galactomannan mixtures in rats" BIOSCIENCE BIOTECHNOLOGY AND BIOCHEMISTRY, vol. 64, no. 10, octobre 2000 (2000-10), pages 2165-2171, XP002420762 ISSN: 0916-8451
- RAKHMANBERDYEVA ET AL: "Carbohydrates and lipids of Gleditsia triacanthos seeds" MEDICINAL & AROMATIC PLANTS ABSTRACTS, NATIONAL INSTITUTE OF SCIENCE COMMUNICATION AND INFORMATION, IN, vol. 25, no. 1, février 2003 (2003-02), XP018006481 ISSN: 0250-4367
- FOUSSARD-BLANPIN O ET AL: "[Pharmacodynamic study of the cardiac effects of triacanthine, a natural derivative of adenine and a synthetic derivative, 7-dimethylaminoethyladenine]" ANNALES PHARMACEUTIQUES FRANÇAISES APR 1969, vol. 27, no. 4, avril 1969 (1969-04), pages 257-259, XP009078930 ISSN: 0003-4509

## Description

La présente invention concerne une nouvelle utilisation dans le domaine cosmétique et/ou dermatologique, et plus particulièrement l'utilisation d'un extrait de Gleditsia et/ou de triacanthine pour préparer une composition destinée à traiter et/ou à prévenir les surcharges adipeuses et la cellulite.

La peau comprend des couches superficielles, à savoir l'épiderme, et des couches plus profondes, le derme et l'hypoderme, et chacune possède des propriétés spécifiques permettant à l'ensemble de réagir et s'adapter aux conditions de son environnement. L'épiderme, qui est composé de trois types de cellules, à savoir des kératinocytes (90.% des cellules épidermiques), des mélanocytes (2 à 3% des cellules épidermiques) et des cellules de Langerhans, constitue la couche externe et joue un rôle fondamental pour assurer la protection et le maintien d'une bonne trophicité. Le derme sert de support à l'épiderme et est principalement constitué de fibroblastes et d'une matrice extracellulaire essentiellement à base de collagène et d'élastine. Les fibres de collagène contribuent à la texture et à la tonicité de la peau, et l'élastine est responsable de son élasticité. D'autres cellules, comme les macrophages et les leucocytes, sont également présentes dans la couche du derme. L'hypoderme, qui est la couche la plus profonde de la peau, contient les adipocytes qui produisent des lipides pour que le tissu sous-cutané fabrique une couche grasse protégeant les muscles, les os, et les organes internes contre les chocs.

Les adipocytes synthétisent des triglycérides par lipogénèse à partir d'acides gras libres et de glycérol provenant de la dégradation du glucose. Les acides gras et le glucose sont apportés à l'organisme par les aliments. Inversement, les triglycérides contenus dans les adipocytes subissent une lipolyse sous l'action d'enzymes et libèrent du glycérol ou des esters de glycérol ainsi que des acides gras qui peuvent à leur tour circuler dans l'organisme et/ou être captés par des adipocytes où ils sont à nouveau transformés en triglycérides par lipogénèse. Ainsi, en cas de déséquilibre entre la lipogénèse et la lipolyse, il peut se produire une accumulation excessive de triglycérides qui se traduit par des surcharges adipeuses, ou surcharges graisseuses localisées.

Les surcharges adipeuses sont des défauts susceptibles de nuire à l'aspect esthétique de l'individu, et elles constituent aussi des états physiologiques anormaux chez l'homme, mais surtout chez la femme, où les accumulations de cellulite sont toujours jugées disgracieuses. Ces états pathologiques, bien que différents de l'obésité, nécessitent généralement un traitement systémique. Bien souvent, à défaut de traitement thérapeutique, un traitement cosmétique est souhaité pour lutter contre les surcharges adipeuses, et pour en atténuer les effets disgracieux.

Aussi, il existe un besoin constant de mettre au point des compositions susceptibles de procurer un effet lipolytique utile dans le traitement des surcharges adipeuses, tant sur les plans cosmétique que thérapeutique. L'effet lipolytique d'une substance peut notamment s'évaluer par le dosage du glycérol libéré dans un milieu de culture d'adipocytes.

De nombreuses substances, notamment d'origine végétale, ont été proposées pour tenter de lutter contre la cellulite et les surcharges adipeuses, et diverses compositions ont été mises au point. Ainsi, des oligomères procyanidoliques dérivés des pépins de raisin, des flavonoïdes, tel que le ruscus, les extraits de vigne rouge, de marron d'inde, d'hamamélis, ont une certaine action de drainage circulatoire. La caféine, et divers dérivés de la caféine, peuvent contribuer à diminuer l'oedème et la rétention d'eau, à augmenter la lipolyse et à limiter le stockage des graisses. Toutefois, la caféine à concentration élevée peut entraîner des effets secondaires, par exemple des palpitations, chez certains sujets. C'est pourquoi des dérivés ou des associations à base de caféine ont été proposés, et par exemple des caféine carboxylates comme dans le brevet FR 2 639 541 et l'association de caféine et d'extraits d'algues comme dans le brevet FR 2 490 492. La demande WO 2006045932 propose d'utiliser de l'huile de chaulmoogra, éventuellement en association avec une base xanthique telle que la caféine, en raison de son effet lipolytique vérifié par son action sur les adipocytes humains en culture.

Le Gleditsia est un arbre de 25 à 30 m de hauteur faisant partie de la famille des Fabacées, originaire d'Amérique du Nord. Ses rameaux sont garnis d'épines simples ou ramifiées, ses feuilles sont caduques, doublement composées de folioles bordées de dents arrondies. Le fruit du Gleditsia est une gousse longue, aplatie en forme de faux et contenant de 10 à 20 graines, se desséchant sur l'arbre avant de tomber.

Les Gleditsia contiennent, dans leurs feuilles ou leurs gousses, une- base purique, la triacanthine, ou N-6-(Δ²-isopentényl)-adénine, qui présente la formule générale suivante :

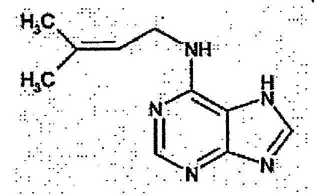

La triacanthine présente une toxicité peu élevée, puisque sa DL50 est de l'ordre de 30 mg/kg, contre 60 mg/kg pour la caféine, autre base purique classique. La triacanthine exerce une action tonicardiaque chez l'animal, décrite par O. Foussard-Blanpin et al., Annales Pharmaceutiques Françaises vol. 27, p. 257-259 (avril 1969).

Outre la triacanthine, les gousses contiennent des flavonoïdes, des saponines (dérivés de catéchine), et les graines contiennent principalement un mucilage riche en arabinose.

Les feuilles et les gousses contiennent environ 100 mg/kg de triacanthine par kg de plante sèche.

Le genre Gleditsia regroupe de nombreuses espèces et variétés existant dans le monde. On peut citer les espèces suivantes : *Gleditsia triacanthos L.* (ou *Gleditsia ferox*), *Gleditsia australis Hemsl.* (encore appelé *Gleditsia fera Merr., Gleditsia sinensis* ou *Gleditsia thorelii*), *Gleditsia africana Walv.* (ou *Gleditsia africanum Harms*.), *Gleditsia japonica Micq., Gleditsia javanica Lam. (ou Parria javanica Merr.), Gleditsia officinalis Hemsl., Gleditsia inermis, Gleditsia amorphoides, Gleditsia aquatica, Gleditsia capsica, Gleditsia delavayi, Gleditsia horrida, Gleditsia koraiensis, Gleditsia macracantha, Geditsia rolfei, Gleditsia texana.*

Dans la pharmacopée africaine, la pulpe de fruit est utilisée pour traiter certaines maladies pulmonaires. Son utilisation pour le traitement du rhume, de la dyspepsie, de la rougeole, de la coqueluche et de certains troubles sanguins a également été reportée. R.K. Rakhmanberdyeva et al., Medicinal and Aromatic Plants Abstracts, vol. 25 n° 1 (Fev. 2003) ont montré que les graines de Gleditsia triacanthos contiennent des galactomannanes, et Y. Yamamoto et al., Bioscience Biotechnology and Biochemistry, vol. 64 n° 10, p. 2165-2171 (oct. 2000) ont montré que des mélanges de galacto-mannanne et de gomme xanthane peuvent avoir un effet hypo-lipidémique chez le rat.

Les études réalisées par la demanderesse ont démontré de manière inattendue que les extraits de Gleditsia et la triacanthine présentent un effet lipolytique vérifié par une action sur les adipocytes humains en cultures, se traduisant par une augmentation du glycérol libéré. Les résultats des tests in vitro mettant en oeuvre ces propriétés sont détaillés ci-après.

La présente invention a donc pour objet une nouvelle utilisation cosmétique d'un extrait de Gleditsia et/ou de triacanthine en une concentration appropriée, destinées au traitement et à la prévention des surcharges adipeuses et de la cellulite.

L'invention a également pour objet l'utilisation d'un extrait de Gleditsia et/ou de la triacanthine pour la préparation d'un médicament à effet lipolytique pour le traitement des surcharges adipeuses et de la cellulite.

L'invention a encore pour objet un procédé de traitement cosmétique de la peau, plus particulièrement pour prévenir ou réduire les surcharges adipeuses et la cellulite, par application d'une composition contenant un extrait de Gleditsia et/ou de la triacanthine sur la zone de la peau nécessitant un tel traitement.

Enfin, l'invention a pour objet l'utilisation combinée d'un extrait de Gleditsia et/ou de triacanthine, et d'un agent lipolytique complémentaire et/ou d'un agent veinotonique pour préparer des compositions cosmétiques et pharmaceutiques destinées au traitement et à la prévention des surcharges adipeuses et de la cellulite, ainsi que des compositions cosmétiques et/ou pharmaceutiques à usage topique contenant une telle combinaison.

L'invention peut notamment permettre la préparation de compositions destinées à améliorer l'ovale du visage, dont le relâchement peut être dû à la présence de surcharges adipeuses.

Plusieurs espèces de Gleditisa peuvent être utilisées dans la présente invention, et par exemple *Gleditsia triacanthos L.* (ou *Gleditsia ferox*), *Gleditsia australis Hemsl.* (encore appelé *Gleditsia fera Merr., Gleditsia sinensis* ou *Gleditsia thorelii*), *Gleditsia africana Walv.* (ou *Gleditsia africanum Harms.*), *Gleditsia japonica Micq., Gleditsia javanica Lam.* (ou *Parria javanica Merr*.), *Gleditsia officinalis Hems., Gleditsia inermis, Gleditsia amorphoides, Gleditsia aquatica, Gleditsia capsica, Gleditsia delavayi, Gleditsia horrida, Gleditsia koraiensis, Gleditsia macracantha, Geditsia rolfei, Gleditsia texana.*

Parmi ces différentes espèces de Gleditsia, l'espèce *Gleditsia triacanthos L.* (févier épineux ou févier d'Amérique) est préférée, car elle est facile d'accès et le ramassage de ses gousses est relativement courant.

Les agents lipolytiques complémentaires peuvent être choisis notamment parmi les bases xanthiques, les extraits d'algues, les silanols, le *Coleus forskohlii,* le *Garcinia cambodgia,* l'huile de chaulmoogra, les guggulipides, un extrait de *Uncaria tomentosa.* Ainsi, l'huile de chaulmoogra, qui est extraite de graines de plantes du genre Hydnocarpus, par exemple des variétés telles que *Hydnocarpus wightiana et Hydnocarpus* anthelmintica, peut être avantageusement associée à l'extrait de Gleditsia suivant la présente invention.

Les bases xanthiques sont constituées par les trois bases puriques, à savoir la caféine, la théophylline et la théobromine. La caféine, la théophylline et la théobromine sont des dérivés méthylés de la xanthine qui diffèrent entre eux par la position et le nombre de groupements méthyles. La théobromine, ou diméthyl-3,7 xanthine, est un alcaloïde extrait de la graine de cacaoyer, *Theobroma cacao* ; on en trouve aussi en faible quantité dans le thé et le café. La théophylline, ou diméthyl-1,3 xanthine, est un alcaloïde extrait des feuilles du théier, *Thea sinensis.* On peut aussi en faire la synthèse par méthylation de la xanthine. La caféine, ou triméthyl-1,3,7 xanthine, est un alcaloïde extrait des feuilles du théier, et des graines de café. La caféine peut également être obtenue à partir des graines du kolatier, du guarana et du maté, ainsi qu'à partir des graines de porangaba (*Cordia salicifolia*) de la famille des boraginées. On peut aussi l'obtenir par synthèse en méthylant la théobromine et la théophylline.

Les extraits d'algues utilisables dans l'invention comme agent lipolytique peuvent être choisis parmi *Gelidium cartilagineum,* des Fucus et des laminaires.

Les silanols peuvent agir en stimulant l'adényl cyclase qui cyclise l'AMP en AMPc, et ils renforcent ainsi l'action lipolytique.

Le *Coleus forskohlii,* utilisé en médecine ayurvédique, stimule l'adényl cyclase au niveau de l'adipocyte, tandis que le *Garcinia cambodgia,* riche en acide hydroxycitrique, limite la synthèse des acides gras et l'accumulation des triglycérides.

Les agents veinotoniques avantageusement utilisés dans les compositions de l'invention en combinaison avec l'extrait de Gleditsia et/ou avec la triacanthine peuvent être choisis parmi les extraits végétaux à saponosides, les extraits végétaux à coumarine et les extraits végétaux à flavonoïdes.

Les plantes à saponosides ont une activité veinotonique importante ainsi que des propriétés anti-inflammatoires et anti-oedémateuses. Elles ont aussi fréquemment des propriétés vasoconstrictives favorisant la circulation capillaire ainsi que des propriétés décongestionnantes améliorant la circulation lymphatique. Ces plantes peuvent être choisies parmi le fragon (*Ruscus aculeatus* dont on extrait la rusco-génine), le lierre (qui fournit l'hédéragénine), le marronnier d'Inde (*Aesculus hippocastanum* dont on extrait l'escine).

Les plantes à coumarine procurent une action anti-oedémateuse, vasculo-protectrice et veinotonique, et peuvent être choisies parmi le marronnier d'Inde (dont on extrait l'esculoside), et le melilot (*Melilotus officinalis,* dont on extrait le mélilotoside qui s'hydrolyse en coumarine).

Les plantes à flavonoïdes ont pour effet de diminuer la fragilité capillaire et de renforcer la résistance des vaisseaux. Elles peuvent être choisies parmi le gingko (*Gingko biloba*) et le *Sophora japonica* dont on peut extraire la troxérutine.

Suivant une forme avantageuse de réalisation, les compositions utilisées dans l'invention contiennent en outre un agent restructurant, qui aide à la restructuration du tissu conjonctif et améliore l'élasticité de la peau.

Les agents restructurants peuvent être choisis parmi les silanols, la *Centella asiatica,* l'hespéritine méthyl chalcone et les alpha-hydroxy acides.

La *Centella asiatica* est un stimulant de la synthèse du collagène et des mucopolysaccharides par les fibroblastes. De plus, il exerce une action positive sur les symptômes de l'insuffisance veineuse chronique des membres inférieurs. L'hespéritine méthyl chalcone possède une action anti-glycation et empêche la rigidification des fibres de collagène entre elles. Les AHA, ou alpha-hydroxy acides, sont connus pour améliorer la pénétration des actifs grâce à leur action kératolytique.

Un activateur de la lipoprotéine lipase peut aussi être incorporé dans les compositions de l'invention.

L'extrait de Gleditsia utilisé dans la présente invention est obtenu par extraction dans l'eau bouillante de gousses ou de feuilles préalablement séchées et broyées, et ajustement du titre à 1 mg/l de triacanthine. L'extrait est stabilisé au butylène glycol. Le dosage est réalisé par HPLC par rapport à un échantillon témoin de triacanthine. L'extrait aqueux obtenu à partir de feuilles de Gleditsia triacanthos possède les caractéristiques analytiques suivantes :
- densité à 22°C _{:} 1,00 - 1,05
- indice de réfraction à 22 °C : 1,34 - 1,36
- teneur en triacanthine : 0,9 - 1,1 mg/l

La teneur en matière sèche est d'environ 1,3% dans le cas de l'extrait de feuilles de Gleditsia, et de 7,45% dans le cas de l'extrait de gousses.

La teneur en extrait de Gleditsia des compositions suivant la présente invention est généralement comprise entre 0,1 et 5%, et leur teneur en triacanthine peut être comprise entre 0,0001 et 2%.

Lorsque l'extrait de Gleditsia et/ou la triacanthine est associé à un agent lipolytique complémentaire, il peut être avantageux d'utiliser un milieu comportant une phase aqueuse présentant une constante diélectrique d'environ 60, par exemple au moyen d'un milieu présentant un degré alcoolique (par exemple éthylique) d'environ 30%.

Les compositions conformes à la présente invention sont de préférence administrables par voie topique. Suivant la terminologie classique, l'administration par voie topique désigne toute méthode consistant à appliquer la substance ou la composition directement sur la peau, sur la zone nécessitant le traitement.

Conformément à la présente invention, la composition administrable par voie topique peut avantageusement contenir, outre les composants de base décrits ci-dessus, une ou plusieurs autres substances connues pour exercer des effets complémentaires bénéfiques pour la peau, et plus particulièrement le tocophérol, la vitamine A (rétinol), l'acide rétinoïque, des agents bactéricides, ainsi que des extraits végétaux connus pour favoriser l'effet d'amincissement comme des extraits huileux de thé vert et de café vert ou un extrait alcoolique de lierre.

Certains de ces extraits, notamment l'extrait glycolique de lierre (hédéragénine), présentent l'avantage de faciliter la pénétration de la composition de l'invention à travers l'épiderme et le derme. D'autres composés connus pour faciliter la pénétration des principes actifs de compositions cosmétiques à travers l'épiderme sont par exemple l'éthoxydiglycol et des saponosides du type hédéragénine.

On peut aussi ajouter avantageusement un composé accélérant la microcirculation et augmentant la température locale, tel que par exemple le nicotinate de méthyle, afin de faciliter l'élimination des glycérides.

Les compositions cosmétiques et pharmaceutiques conformes à la présente invention, sont destinées de préférence à une administration topique, et contiennent donc des supports et excipients couramment utilisés dans des compositions de ce type telles que des émulsions H/E ou E/H, des crèmes, des gels, des laits, des patches ou des lotions. Dans le cas des émulsions, la phase grasse peut représenter entre 10 eL 60% environ du poids de la composition, la phase aqueuse entre 10 et 80% environ et l'agent émulsionnant entre 2 et 20%, le reste étant constitué par les composants de base indiqués ci-dessus et les autres composants mentionnés ci-après.

La composition peut encore contenir diverses substances et excipients choisis en fonction de leurs propriétés connues et de la forme galénique envisagée. Ainsi, on peut incorporer dans la composition des conservateurs, des agents émulsionnants, des agents viscosants, des épaississants, des gélifiants, des antioxydants, des agents hydratants, des tensioactifs, des parfums, des huiles, des lipides, un solvant spécifique ainsi que de l'eau et divers additifs destinés à améliorer les propriétés physiques de la composition.

On peut choisir l'agent émulsionnant parmi des polymères carboxyvinyliques à haut poids moléculaire (par exemple le Carbopol^{®}), des polysorbates (par exemple le Tween 20^{®} ou le Polysorbate 80^{®}), des esters de sorbitan et en particulier un monostéarate, un tristéarate, un monopalmitate, et un laurate de sorbitan. On peut encore utiliser d'autres agents émulsionnants tels que divers dérivés d'acide stéarique ou palmitique, et par exemple le stéarate de PEG 100^{®}, des mono- ou diglycérides d'acide stéarique ou palmitique, un stéarate de propylène glycol auto-émulsionnable, ou encore le polyglycéryl-2-sesquioléate, l'éther cétylique de polyoxyéthylène, un polyglucoside de siloxane, ou une silicone émulsionnable. On peut encore utiliser des mélanges émulsionnants non ioniques tels que le Protegin X^{®}.

Les agents viscosants utilisés dans les compositions de l'invention peuvent être choisis parmi divers polymères d'acide acrylique, une gomme cellulose, une silice, des polymères carboxyvinyliques, un silicate d'aluminium et de magnésium, et on peut utiliser par exemple la silice colloïdale vendue sous la marque Aerosil 200^{®} ou un acide polyacrylique réticulé tel que le Carbopol 940^{®}.

Les gélifiants ou épaississants peuvent être choisis par exemple parmi les polyacrylamides, des acrylates comme le Pemulen^{®}, les dérivés de cellulose comme l'hydroxypropyl cellulose, ou les gommes naturelles.

Les agents hydratants utilisés peuvent être choisis par exemple parmi un polyol, le sorbitol, le maltitol, le pentaérythritol, les polyacrylates et polyméthacrylates de glycéryle, le glycérol ou des dérivés de glycérol. On peut aussi ajouter des émollients tels qu'un malate d'alkyle, l'isohexadécane, des triglycérides d'acide caprique ou caprylique, etc.

Les conservateurs usuels de la technique des compositions dermatologiques ou cosmétologiques peuvent être utilisés dans l'invention, et par exemple l'acide benzoïque et un p-hydroxybenzoate d'alkyle tel que les p-hydroxybenzoates de méthyle et de propyle (Méthylparaben et Propylparaben), un alcool tel que le phénoxy-éthanol ou encore la chlorphénésine ou l'imidazolidinyl urée.

Les constituants de la phase grasse, c'est-à-dire les huiles et lipides, peuvent être choisis parmi l'huile de jojoba, l'huile de maïs, l'huile de vaseline, l'huile de coco hydrogénée, l'huile de carthame, des glycérides d'acides gras saturés, l'acide stéarique, l'acide palmitique, le stéarate d'octyle, le palmitate de glycéryle, le palmitate d'octyle, un triglycéride d'acides caprique et caprylique, le 2-octyldodécanol, le polyéthylène glycol, l'adipate d'éthyl-2 hexyle, ou encore des huiles de silicones telles que le méthyl phényl polysiloxane, la diméthicone, la cyclométhicone, la cyclométhicone/diméthicone copolyol, la phényl diméthicone.

La composition peut aussi contenir un solvant choisi en fonction des composants utilisés et de la forme d'administration envisagée. Le solvant peut être par exemple de l'eau, et de préférence de l'eau déminéralisée, ou un solvant spécifique tel que le propylène glycol, un éther de diéthylène glycol, ou un alcool, en particulier l'éthanol.

Le pH de la composition est généralementcompris entre 5 et 7,5, de préférence entre 5,5 et 6,5, et il peut être ajusté, selon les compositions, par addition d'un acide tel que l'acide citrique ou d'une base telle que l'hydroxyde de sodium.

La composition conforme à la présente invention peut être présentée sous les formes classiquement utilisés pour une application topique, c'est-à-dire sous forme de gel, lotion, émulsion (en particulier crème ou lait), patchs transdermiques, masque ou pommade, contenant des excipients et supports usuels compatibles et pharmaceutiquement acceptables. Ces formes d'administration par voie topique sont préparées par les techniques connues, et par exemple, dans le cas d'une crème, par dispersion d'une phase grasse dans une phase aqueuse pour obtenir une émulsion huile dans eau, ou inversement pour préparer une émulsion eau dans huile. Dans le cas de crèmes, on préfère utiliser des émulsions à structure lamellaire contenant peu de produits éthoxylés ou n'en contenant pas du tout.

A titre d'exemple, on peut préparer des compositions topiques conformes à l'invention sous forme de crèmes amincissantes, de laits ou de gels amincissants, utilisables en une ou plusieurs applications quotidiennes, la durée du traitement pouvant être généralement de l'ordre de un à trois mois.

Les exemples qui suivent illustrent la présente invention, sans en limiter la portée.

Des exemples de formulations de compositions à base d'extraits de Gleditsia ou de triacanthine sont rapportés aux exemples 1 à 3. Dans ces exemples, les parties sont exprimées en poids, sauf indication contraire.

L'exemple 4 est relatif à des tests destinés à évaluer la tolérance d'un extrait de Gleditsia sur des épidermes reconstitués SKINETHIC^{™}, et l'exemple 5 décrit des essais permettant d'évaluer l'effet lipolytique d'un extrait de Gleditsia par le relargage du glycérol dans un milieu de culture d'adipocytes humains.

### Exemple 1

Un gel amincissant à base d'extrait de Gleditsia ayant la composition indiquée ci-après est préparé suivant les techniques usuelles.

| | |
|---|---|
| Sodium heptonate | 0,1 |
| Glycérine | 2,0 |
| Octyldodécanol | 2,0 |
| Polymère carboxyvinylique | 0,5 |
| Oléate de décyle | 2,0 |
| Copolymère acrylate de sodium/laurate de diméthyle acryloyle | 0,5 |
| Trométhamine | 0,6 |
| Phényl diméthicone | 1,0 |
| Extrait glycolique de Gleditsia | 5,0 |
| Extrait de laminaria digitata | 7,0 |
| Extrait de Coléus | 3,0 |
| Parfum | 0,1 |
| Conservateurs | 0,6 |
| Eau déminéralisée QSP | 100,0 |

### Exemple 2

Une crème amincissante à base d'extrait de Gleditsia ayant la composition indiquée ci-après est préparée suivant les techniques usuelles.

| | |
|---|---|
| Glycérine | 3,0 |
| Pentylène glycol | 5,0 |
| Capryl glycol | 3,0 |
| Sodium heptonate | 0,1 |
| Gomme xanthane | 0,2 |
| Octyl glycérol | 0,3 |
| Caprylyl glycocolle | 1,5 |
| Stéarate d'acétylglycol | 0,8 |
| Cétéaryl glucoside | 4,0 |
| Alcool cétylique | 1,0 |
| Tristéarine | 0,8 |
| Phényltriméthicone | 1,0 |
| Cyclopentasiloxane | 4,0 |
| Tocophérol | 0,7 |
| Copolymère acrylate de sodium/laurate de diméthyl acryloyl | 0,5 |
| Isohexadécane | 0,3 |
| Polysorbate 80 | 0,8 |
| Extrait glycolique de lierre | 2,0 |
| Extrait glycolique de Gleditsia | 3,0 |
| Théophylline | 1,0 |
| Parfum | 0,1 |
| Eau déminéralisée QSP | 100,0 |

### Exemple 3

Un gel ou un lait amincissant à base de triacanthine ayant la composition indiquée ci-après est préparé suivant les techniques usuelles.

| | |
|---|---|
| Triacanthine | 0,5 |
| Sodium heptonate | 0,1 |
| Pentylène glycol | 5,0 |
| Capryl glycol | 3,0 |
| Octyl glycérol | 0,3 |
| Chlorhexidine digluconate | 0,2 |
| Octyl dodecanol | 2,0 |
| Polymère carboxyvinylique | 0,5 |
| Triglycéride caprique/caprylique | 3,0 |
| Diméthylisosorbide | 1,0 |
| Trométhamine | 0,6 |
| Copolymère acrylate de sodium/laurate de diméthylacryloyle | 0,5 |
| Phényldiméthicone | 1,0 |
| Extrait glycolique de lierre | 4,0 |
| Ruscogénines | 0,1 |
| Théobromine | 0,5 |
| Parfum | 0,2 |
| Eau déminéralisée QSP | 100,0 |

### Exemple 4

Le but de l'étude décrite ci-dessous est d'évaluer la cytotoxicité d'un extrait de Gleditsia sur des épidermes reconstitués, modèle SKINETHIC^{™}, en étudiant la viabilité cellulaire.

### 4-1. Protocole expérimental

Des kératinocytes d'origine humaine sont ensemencés sur des filtres en polycarbonate de 0,5 cm² dans un milieu défini (MCDB 153 modifié) et supplémenté. Les cellules sont cultivées pendant 14 jours à l'interface air/liquide, le milieu de culture étant changé tous les deux jours. Les épidermes ainsi formés sont utilisés pour la réalisation de l'étude à partir du 17^{e} jour de la culture.

L'essai est conduit en duplicate à chaque temps expérimental après 24 heures de traitement.

La constitution des lots est la suivante :
- Lot 1 : deux épidermes témoins ne recevant aucun produit.
- Lot 2 : deux épidermes traités recevant une composition sans extrait de Gleditsia.
- Lot 3 _{:} deux épidermes traités recevant la même composition contenant un extrait de Gleditsia à 5%.

La viabilité cellulaire est d'abord étudiée par réaction colorimétrique au sel de tétrazonium (MTT).

Le sel de tétrazolium a la propriété d'être réduit en cristaux bleux de formazan par la succinate déshydrogénase mitochondriale des cellules. Cet enzyme, qui joue un rôle important dans le cycle de Krebs, catalyse la déshydrogénation du succinate en fumarate. C'est l'activité de cet enzyme, flavoprotéine très fortement liée à la membrane interne mitochondriale qui est mesurée, par réduction du MTT. Par un dosage spectrophotométrique, il est possible de déterminer la toxicité d'une population cellulaire donnée. En effet, l'absorbance est directement reliée à l'activité des succinates déshydrogénases, elle-même liée à la toxicité cellulaire.

0,15 ml de milieu de culture contenant 10% vol/vol de solution de MTT est pipetté sous chacun des filtres/support de culture. Après une incubation de 30 minutes à température ambiante, la couleur des différentes cultures est observée et notée. Les cultures doivent être de couleur bleue/pourpre intense, preuve de la viabilité des cellules de l'épiderme.

La viabilité cellulaire est également étudiée par examen histologique, en utilisant les lots 1 à 3 dont la composition a été indiquée ci-dessus. Pour cela, les épidermes fixés dans une solution contenant 10% de formaldéhyde sont inclus dans des blocs en paraffine. Les coupes verticales de 4 microns sont colorées à l'hématoxyline/éosine et photographiées sous un microscope optique.

Les cultures doivent présenter des couches cellulaires basales, spineuses, granuleuses et cornées intactes, orthokératosiques, et la stratification épidermique doit être régulière et normale. Les cellules de la couche basale doivent être polarisées verticalement. De nombreux grains de kératohyaline doivent être visibles (en violet) dans la couche granuleuse juste sous la couche cornée.

### 4.2.Résultats

L'évaluation de la cytotoxicité par réaction colorimétrique au sel de tétrazonium (MTT) est réalisée en fonction des différentes concentrations de l'extrait de Gleditsia. Les résultats sont regroupés dans le tableau ci-dessous.

| | Densité optique | % |
|---|---|---|
| Témoin | 0,990±0,03 | - |
| Sans Gleditsia | 1,015±0,05 | n.s. |
| Gleditsia (5%) | 0,995±0,07 | n.s. |

| | | |
|---|---|---|
| *n.s.* : *non significatif* | | |

Les épidermes reconstitués traités par les différentes concentrations de l'extrait de Gleditsia à raison de 2 µl/cm² montrent une couleur bleue/pourpre intense, preuve de la viabilité des cellules de l'épiderme.

Par examen histologique, il apparaît que l'extrait de Gleditsia déposé à raison de 2 µl/cm² sur des épidermes reconstitués traités pendant 24 heures, comparativement à des épidermes témoins, n'a induit aucune toxicité. Les images histologiques, après coloration HES, d'épidermes traités par les produits sont comparables à celles des épidermes témoins et répondent aux paramètres histologiques normaux.

Les cultures présentent des couches cellulaires basales, spineuses, granuleuses et cornées intactes, et la stratification épidermique est régulière et normale. Les cellules de la couche basale sont polarisées verticalement. De nombreux grains de kératohyaline sont visibles dans la couche granuleuse juste sous la couche cornée.

En conclusion, dans les conditions expérimentales retenues, l'extrait de Gleditsia ne présente pas de cytotoxicité en comparaison avec le témoin non traité.

### Exemple 5

L'efficacité de l'extrait de Gleditsia suivant la présente invention a été vérifiée par des tests d'évaluation de l'effet lipolytique par relargage du glycérol dans un milieu de culture d'adipocytes humains. Cette activité est en effet significative d'un effet lipolytique se traduisant par une hydrolyse des lipides totaux en glycérol et en acides gras libres.

### 5.1. Préparation des adipocytes humains en culture

Les adipocytes sont obtenus par prélèvement du tissu adipeux à partir d'une chirurgie plastique (peau entière), conservé à température ambiante dans un milieu de conservation approprié (au maximum 2 heures après l'opération) puis découpé finement et placé dans un tampon HBSS à pH 7,4. Une solution de collagénase de type II (1 mg/ml dans le tampon HBSS à pH 7,4) est ajoutée au tube contenant les fragments de tissu adipeux (4 volumes de collagénase pour 1 volume de tissu) et l'ensemble est incubé à 37°C pendant 40 à 60 minutes sous agitation constante, sans dioxyde de carbone. Cette première étape permet de libérer les adipocytes de la gangue conjonctive et du stroma vasculaire.

Après incubation, les cellules sont récupérées par filtration sur un filtre en nylon (diamètre 250 µm) et les adipocytes ainsi isolés sont séparés par flottation. Après élimination du sous-nageant tampon HBSS + collagénase) par aspiration, la suspension adipocytaire est lavée par un tampon isotonique KBR pour éliminer toute trace de collagénase ainsi que les substances accumulées au cours de la digestion. En fin de lavage, on obtient une suspension homogène d'adipocytes isolés dans un tampon KBR. Après addition de 3,5% de BSA, le pH est ajusté à 7,5 et le tampon est filtré (filtre 0,22 µm).

La composition du tampon KBR est la suivante (pour 1 litre) :
- NaCl 7,183 g/l
- KCl 0,466 g/l
- CaCl₂, 2H₂O 0,187 g/l
- MgSO₄ 0,144 g/l
- NaH₂PO₄ 0,144 g/l
- NaH₂PO₃ 2,184 g/l
- Glucose 0,826 g/l.

### 5.2. Constitution des lots

L'étude est réalisée en triplicate.

La constitution des lots est la suivante :
- Lot 1 _{:} témoin négatif non traité
- Lot 2 _{:} témoin positif traité par la caféine à 0,4%
- Lots 3 à 5 _{:} traités par un extrait de Gleditsia aux concentrations respectives de 0,2%, 0,5% et 1%.

### 5.3 Dosage enzymatique du glycérol

Le dosage enzymatique du glycérol consiste à doser l'oxydation de la forme réduite du nicotinamide-adénine-dinucléotide (NADH) par mesure de la densité optique (DO) à 385 nm. En effet, la quantité de NADH oxydée au cours de la réaction de conversion du glycérol en pyruvate puis en lactate est proportionnelle à la concentration totale du glycérol.

Sous l'action de trois enzymes (glycérokinase, pyruvate kinase et lactate déshydrokinase) les trois réactions suivantes se produisent : 1) le glycérol réagit avec l'ATP pour donner le glycérol-3-phosphate et l'ADP ; 2) l'ADP réagit avec le phosphoénolpyruvate pour libérer du pyruvate et de l'ATP ; 3) enfin, le pyruvate réagit avec la NADH-H⁻ pour procurer le lactate-NAD.

On utilise un tampon glycilglycine (pH 7,4) contenant NADH (0,83 mg), ATP (2 mg), sulfate de magnésium (1 mg) et phosphoénolpyruvate (1 mg). La suspension enzymatique est constituée de pyruvate kinase (240 u) et de lactate déshydrogénase (220 u). La glycérokinase (34 u) est utilisée en suspension.

La méthode consiste à mélanger dans un premier temps tous les éléments des réactions 2) et 3) ci-dessus, en présence et en l'absence des milieux de culture des adipocytes (avec et sans le produit à tester), et de mesurer la DO (A₁), et dans un deuxième temps à démarrer la réaction 1) en ajoutant la glycérokinase. La DO résultante est alors mesurée à 385 nm (A₂). La différence de DO est obtenue à partir des différences de DO pour le produit à tester d'une part, et pour le témoin d'autre part.

Les résultats obtenus sont présentés dans le tableau ci-dessous.

| | glycérol (mg/l) | % d'augmentation |
|---|---|---|
| Témoin négatif | 42,7 ± 3,9 | |
| Témoin positif (caféine à 0,4%) | 56,9 ± 2,7^{**} | + 33% |
| Extrait de Gleditsia à 0,2% | 42,5 ± 8,2 | - |
| Extrait de Gleditsia à 0,5% | 49,2 ± 3,5^{*} | + 15% |
| Extrait de Gleditsia à 1% | 50,0 ± 2,4^{*} | + 17% |

| | | |
|---|---|---|
| *^{*}significativement différent par rapport au témoin p ≤ 0,05* ***significativement différent par rapport au témoin p ≤ 0,01* | | |

Ces résultats montrent que le traitement des adipocytes humains en culture traités par un extrait de Gleditsia aux concentrations de 0,5% et 1% augmente significativement le taux de glycérol relargué dans le milieu de culture, respectivement de 15 et 17%, comparativement à la culture témoin, la caféine prise comme référence positive augmentant ce taux de 33%.

Il est à noter que le produit utilisé contient 0,5 mg/l de triacanthine. Ainsi, le produit « extrait de Gleditsia » à 1% contient seulement 0,0005 mg/l de triacanthine, et il apparaît que ce produit donne un résultat de glycolyse représentant environ 50% du résultat obtenu avec l'échantillon contenant 400 mg/l de caféine, c'est-à-dire une quantité de caféine bien plus élevée. La triacanthine, et par conséquent l'extrait de Gleditsia qui la contient, sont donc particulièrement efficaces pour l'utilisation considérée.

## Revendications

1. Utilisation cosmétique d'un extrait de Gleditsia et/ou de triacanthine destinée au traitement et/ou à la prévention des surcharges adipeuses et de la cellulite.

2. Utilisation selon la revendication 1, **caractérisée en ce que** l'extrait de Gleditsia est un extrait de Gleditsia triacanthos L.

3. Utilisation selon la revendication 1 ou 2, **caractérisée en ce que** l'extrait est obtenu à partir de feuilles ou de gousses de Gleditsia.

4. Utilisation selon la revendication 3, **caractérisée en ce que** l'extrait est obtenu par extraction dans un mélange butylène glycol/eau.

5. Utilisation selon l'une quelconque des revendications 1 à 4, **caractérisée en ce que** l'extrait aqueux de Gleditsia triacanthos possède les caractéristiques analytiques suivantes :
- densité à 22°C _{:} 1,00 - 1,05
- indice de réfraction à 22 °C . 1,34 - 1,36
- teneur en triacanthine : 0,9 - 1,1 mg/l

6. Utilisation selon la revendication 1, **caractérisée en ce que** la composition comprend entre 0,1 et 5% en poids d'extrait de Gleditsia.

7. Utilisation selon la revendication 1, **caractérisée en ce que** la composition comprend entre 0,0001 et 2% en poids de triacanthine.

8. Utilisation d'un extrait de Gleditsia et/ou de triancanthine pour la préparation d'un médicament à effet lipolytique pour le traitement des surcharges adipeuses.

9. Utilisation d'un extrait de Gleditsia et/ou de triacanthine en combinaison avec un agent lipolytique complémentaire et/ou un agent veinotonique pour préparer une composition pharmaceutique destinée au traitement et à la prévention des surcharges adipeuses.

10. Composition pharmaceutique à usage topique destinée au traitement et à la prévention des surcharges adipeuses, **caractérisée en ce qu'**elle comprend un extrait de Gleditsia et/ou de la triacanthine, en combinaison avec un agent lipolytique et/ou un agent veinotonique.

11. Composition selon la revendication 10, **caractérisée en ce que** l'agent lipolytique complémentaire est choisi parmi les bases xanthiques, les extraits d'algues, les silanols, le *Coleus forskohlii,* le *Garcinia cambodgia,* l'huile de chaulmoogra, les guggulipides, un extrait de *Uncaria tomentosa.*

12. Composition selon la revendication 10, **caractérisée en ce que** l'agent veinotonique est choisi parmi les extraits végétaux à saponosides, les extraits végétaux à coumarine, et les extraits végétaux à flavonoïdes.

13. Composition selon l'une quelconque des revendications 10 à 12, **caractérisée en ce qu'**elle contient en outre un agent restructurant.

14. Procédé de traitement cosmétique de la peau, pour prévenir ou réduire les surcharges adipeuses et la cellulite, par application d'une composition contenant un extrait de Gleditsia et/ou de la triacanthine sur la zone de la peau nécessitant un tel traitement.

## Claims

1. The cosmetical use of an extract of Gleditsia and/or triacanthine for the treatment and/or prevention of excess adipose tissue and cellulite.

2. The use according to claim 1, **characterized in that** the extract of Gleditsia is an extract of Gleditsia triacanthos L.

3. The use according to claim 1 or 2, **characterized in that** the extract is obtained from leaves or seed-pods of Gleditsia.

4. The use according to claim 3, **characterized in that** the extract is obtained by extraction in a butylene glycol/water mixture.

5. The use according to any one of claims 1 to 4, **characterized in that** the aqueous extract of Gleditsia triacanthos has the following analytical characteristics:
- density at 22°C _{:} 1,00 - 1,05
- refraction index at 22 °C : 1,34 - 1,36
- triacanthine content : 0,9 - 1,1 mg/l

6. The use according to claim 1, **characterized in that** the composition comprises from 0,1 to 5% by weight of Gleditsia extract.

7. The use according to claim 1, **characterized in that** the composition comprises from 0,0001 to 2% by weight of triacanthine.

8. The use of an extract of Gleditsia and/or triancanthine for the preparation of a medicament having lipolytic effect for the treatment of excess adipose tissue.

9. The use of an extract of Gleditsia and/or triancanthine in combination with a complementary lipolytic agent and/or a veinotonic agent for preparing a pharmaceutical composition for the treatment and the prevention of excess adipose tissue.

10. Pharmaceutical composition for topical use for the treatment and the prevention of excess adipose tissue, **characterized in that** it comprises an extract of Gleditsia and/or triacanthine, in combination with a lipolytic agent and/or a veinotonic agent.

11. Composition according to claim 10, **characterized in that** the complementary lipolytic agent is selected from the xanthic bases, the algae extracts, the silanols, *Coleus forskohlii, Garcinia cambodgia,* chaulmoogra oil, guggulipids, a *Uncaria tomentosa extract*.

12. Composition according to claim 10, **characterized in that** the veinotonic agent is selected from the saponosides vegetal extracts, the coumarine vegetal extracts, and the flavonoides vegetal extracts.

13. Composition according to any one of claims 10 to 12, **characterized in that** it further contains a restructuring agent.

14. Method for the cosmetic treatment of the skin, for preventing or reducing the excess adipose tissue and cellulite, by applying a composition containng an extract of Gleditsia and/or triacanthine on the area of the skin in nedd of such a treatment.

## Patentansprüche

1. Kosmetische Verwendung eines Gleditsiaextrakts und/oder von Triacanthin zur Behandlung und/oder Prävention von überschüssigen Fetteinlagerungen und Cellulitis.

2. Verwendung nach Anspruch 1, **dadurch gekennzeichnet, dass** der Gleditsiaextrakt ein Extrakt von Gleditsia triacanthos L ist.

3. Verwendung nach Anspruch 1 oder 2, **dadurch gekennzeichnet, dass** der Extrakt ausgehend von Gleditsiablättern oder -hülsen erhalten wird.

4. Verwendung nach Anspruch 3, **dadurch gekennzeichnet, dass** der Extrakt durch Extraktion in einem Butylenglykol/Wasser-Gemisch erhalten wird.

5. Verwendung nach einem der Ansprüche 1 bis 4, **dadurch gekennzeichnet, dass** der wässrige Extrakt von Gleditsia triacanthos folgende Analyseeigenschaften aufweist:
- Dichte bei 22 °C: 1,00 - 1,05
- Brechungsindex bei 22 °C: 1,34 - 1,36
- Triacanthingehalt: 0,9 - 1,1 mg/l.

6. Verwendung nach Anspruch 1, **dadurch gekennzeichnet, dass** die Zusammensetzung 0,1 bis 5 Gew.-% Gleditisiaextrakt umfasst.

7. Verwendung nach Anspruch 1, **dadurch gekennzeichnet, dass** die Zusammensetzung 0,001 bis 2 Gew.-% Triacanthin umfasst.

8. Verwendung eines Gleditsiaextrakts und/oder von Triacanthin zur Herstellung eines Medikaments mit lipolytischer Wirkung zur Behandlung von überschüssigen Fetteinlagerungen.

9. Verwendung eines Gleditsiaextrakts und/oder von Triacanthin in Kombination mit einem ergänzenden lipolytischen Mittel und/oder einem Venentonikum zur Herstellung einer kosmetischen oder pharmazeutischen Zusammensetzung zur Behandlung und Prävention von überschüssigen Fetteinlagerungen.

10. Pharmazeutische Zusammensetzung zur topischen Anwendung zur Behandlung und Prävention von überschüssigen Fetteinlagerungen, **dadurch gekennzeichnet, dass** sie einen Gleditsiaextrakt und/oder Triacanthin in Kombination mit einem lipolytischen Mittel und/oder einem Venentonikum umfasst.

11. Zusammensetzung nach Anspruch 10, **dadurch gekennzeichnet, dass** das ergänzende lipolytische Mittel aus Xanthinbasen, Algenextrakten, Silanolen, Coleus forskohlii, Garcinia cambodgia, Chaulmoograöl, Guggulipiden und einem Extrakt von Uncaria tomentosa ausgewählt ist.

12. Zusammensetzung nach Anspruch 10, **dadurch gekennzeichnet, dass** das Venentonikum aus pflanzlichen Saponosidextrakten, pflanzlichen Cumarinextrakten und pflanzlichen Flavonoidextrakten ausgewählt ist.

13. Zusammensetzung nach einem der Ansprüche 10 bis 12, **dadurch gekennzeichnet, dass** sie außerdem ein restrukturierendes Mittel enthält.

14. Verfahren zur kosmetischen Behandlung der Haut zur Prävention oder Reduktion von überschüssigen Fetteinlagerungen und Cellulitis durch Auftragen einer Zusammensetzung, die einen Gleditisiaextrakt und/oder Triacanthin enthält, auf den Bereich der Haut, der einer solchen Behandlung bedarf.
